(19) Europäisches Patentamt

European Patent Office

Office européen des brevets

(11) **EP 1 141 701 B1**

(12) **FASCICULE DE BREVET EUROPEEN**

(45) Date de publication et mention
de la délivrance du brevet:
**25.02.2004 Bulletin 2004/09**

(21) Numéro de dépôt: **99958248.9**

(22) Date de dépôt: **06.12.1999**

(51) Int Cl.⁷: **G01N 33/10**, G01N 33/483,
G01N 33/02, G01N 21/35

(86) Numéro de dépôt international:
**PCT/FR1999/003025**

(87) Numéro de publication internationale:
**WO 2000/034772 (15.06.2000 Gazette 2000/24)**

(54) **PROCEDE DE SUIVI D'UNE CULTURE DE PLANTES DE GRANDES CULTURES A EPIS**

VERFAHREN ZUM ÜBERWACHEN EINER AHRENPFLANZENKULTUR

METHOD FOR MONITORING PLANT GROWTH IN PANICLE CROPS

(84) Etats contractants désignés:
**AT BE CH CY DE DK ES FI FR GB GR IE IT LI LU
MC NL PT SE**

(30) Priorité: **07.12.1998 FR 9815426**

(43) Date de publication de la demande:
**10.10.2001 Bulletin 2001/41**

(73) Titulaire: **ULICE
F-63200 Riom (FR)**

(72) Inventeurs:
 • **OLLIVIER, Erwan
 F-63530 Volvic (FR)**
 • **VIALLIS, Bruno
 F-63670 Le Cendre (FR)**
 • **MESSAGER, Arnaud
 F-63200 Riom (FR)**

(74) Mandataire: **Blot, Philippe Robert Emile et al
c/o Cabinet Lavoix,
2, place d'Estienne d'Orves
75441 Paris Cedex 09 (FR)**

(56) Documents cités:
 **EP-A- 0 388 082      FR-A- 2 737 573**

 • **PATENT ABSTRACTS OF JAPAN vol. 098, no.
 005, 30 avril 1998 (1998-04-30) & JP 10 002854 A
 (ISEKI &AMP;CO LTD), 6 janvier 1998
 (1998-01-06)**
 • **PATENT ABSTRACTS OF JAPAN vol. 096, no.
 005, 31 mai 1996 (1996-05-31) & JP 08 015141 A
 (SATAKE ENG CO LTD), 19 janvier 1996
 (1996-01-19)**
 • **PATENT ABSTRACTS OF JAPAN vol. 099, no.
 002, 26 février 1999 (1999-02-26) & JP 10 300665
 A (SATAKE ENG CO LTD), 13 novembre 1998
 (1998-11-13)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne un procédé de suivi d'une culture de plantes de type "grandes cultures". à épis

Parmi les plantes de « grandes cultures » à épis, on peut citer les céréales (blé, seigle, orge, avoine, maïs, etc).

Les méthodes d'allotement actuelles nécessitent la maîtrise de la qualité des produits récoltés. Cette maîtrise passe notamment par la connaissance de leur teneur en certains constituants déterminés, notamment en protéines et ce, de manière prévisionnelle, alors que les plantes sont en cours de croissance et qu'elles n'ont pas atteint une maturité suffisante pour être récoltées.

Les organismes collecteurs-stockeurs s'engagent aujourd'hui à fournir des produits dont ils garantissent entre autres critères, l'homogénéité et la teneur en certains constituants. La maîtrise de ces critères passe notamment par un suivi strict de l'évolution des cultures et par différentes mesures prévisionnelles à certains stades de croissance des plantes.

Dans le cas particulier des céréales, et notamment du blé ou de l'orge, il est connu de procéder à une analyse du grain immature une quinzaine de jours avant la récolte, en vue de déterminer la teneur de celui-ci en protéines. Cette information permet d'évaluer par extrapolation la teneur en protéines des grains au moment de la récolte puisqu'elle reste sensiblement identique. Cette information est particulièrement intéressante puisqu'elle correspond à une exigence formulée par les acheteurs de céréales.

Parmi les céréales d'intérêt, on peut citer notamment les céréales de la famille des graminées, telles que le blé, le seigle, l'orge, l'avoine et le maïs.

Cette analyse prévisionnelle facilite en particulier le processus ultérieur de collecte puis d'allotement. Cette dernière opération consiste en la constitution de lots possédant des caractéristiques homogènes.

Les procédés actuels de mesure de la teneur en protéines prévoient la mise en oeuvre d'une analyse sur des échantillons constitués des grains seuls. Ces grains sont issus d'épis préalablement séchés et battus.

En particulier, FR-2.737.573 décrit un procédé et un dispositif d'analyse par infrarouge d'un échantillon dans le domaine céréalier. L'échantillon considéré n'est pas précisé.

De même, EP-A-0.388.082 décrit l'analyse de grains pour en déterminer la teneur notamment en protéines par spectrométrie dans le proche infrarouge. Cette analyse est réalisée directement sur des grains.

Ainsi, la mesure de la teneur en un constituant nécessite un protocole opératoire difficile à mettre en oeuvre pour un agriculteur, puisqu'il convient, pour la préparation de l'échantillon, de couper des épis de céréales, de procéder au séchage de ceux-ci, puis d'en extraire les grains.

Par ailleurs et bien avant la récolte, le suivi de l'état des cultures , et particulièrement l'état de nutrition azoté, permet de mieux conduire les interventions au champ et par conséquent d'atteindre l'optimum de rendement et de qualité du produit récolté. C'est pourquoi différentes méthodes sont aujourd'hui utilisées pour réaliser des diagnostics de l'état de nutrition des plantes ; celles-ci sont encore lourdes à mettré en oeuvre puisqu'elles nécessitent l'extraction du jus des tiges, leur analyse en laboratoire, etc. Il n'existait pas, jusqu'ici, de méthode simple, utilisant les résultats obtenus par les mesures de la teneur en un constituant déterminé dans le domaine du proche infrarouge, directement sur des plantes en développement post-germination et permettant de piloter la fertilisation, notamment azotée ou soufrée.

L'invention a pour but de fournir un procédé de suivi de la culture de plantes de "grandes cultures" par mesure de la teneur en un constituant déterminé, notamment en protéines, ne présentant pas les inconvénients mentionnés ci-dessus et qui soit simple à mettre en oeuvre, notamment par un responsable de silo.

A cet effet, l'invention a pour objet un procédé de suivi d'une culture de plantes de "grandes cultures" à épis incluant une mesure de la teneur en un constituant déterminé par spectrométrie dans le domaine du proche infrarouge directement sur les plantes en développement post-germination, caractérisé en ce que la mesure par spectrométrie est réalisée sur les épis entiers de la plante afin de déterminer la teneur des grains en un constituant déterminé, et le résultat de l'analyse des épis entiers est traité pour tenir compte de la présence des parties végétales différentes des grains.

Selon des modes particuliers de réalisation, le procédé comporte l'une ou plusieurs des caractéristiques suivantes :

- la mesure de la teneur en un constituant déterminé comporte une mesure du spectre d'absorbance en transmission et/ou en réflexion des épis ;

- avant la mesure par spectrométrie, les épis sont répartis par couches, dans chacune desquelles les épis sont disposés parallèlement les uns aux autres, le sens des épis étant alterné d'une couche adjacente à l'autre ;

- la mesure de la teneur en un constituant déterminé comporte une mesure du spectre de réflectance des épis éclairés par le rayonnement solaire naturel ;

- les épis sont portés par une céréale sur pied lors de la réalisation de la mesure ;

- la mesure par spectrométrie est réalisée sur les parties aériennes des plantes afin de calculer l'indice de nutrition azoté dans le but de contrôler et de déterminer l'apport en agents fertilisants nécessaires à une croissance satisfaisante des plantes ;

- la mesure par spectrométrie est réalisée sur les parties aériennes des plantes afin d'estimer l'état de nutrition azoté ou soufré, le déficit hydrique, la carence en oligo-éléments, et / ou l'état sanitaire dans le but de contrôler et de déterminer l'apport en agents fertilisants et/ou en produits phytosanitaires (fongicides, insecticides, etc) nécessaires à une croissance satisfaisante des plantes
- la teneur en le constituant donné est la teneur en protéines, en sucres solubles, en cellulose, en azote, en matières grasses, et/ou en soufre,

Par ailleurs, l'invention concerne un procédé de mesure de la teneur en un constituant déterminé dans des grains issus de céréales, du type comportant :

la préparation d'un échantillon comprenant lesdits grains ;
l'analyse dudit échantillon pour déterminer la teneur des grains en le constituant déterminé ;

caractérisé en ce que l'échantillon, représentatif de la parcelle étudiée, est constitué d'un ensemble d'épis entiers contenant des grains ; et
en ce que la détermination de la teneur des grains en le constituant déterminé comporte une étape de traitement du résultat de l'analyse de l'échantillon pour tenir compte de la présence dans les épis de parties végétales différentes des grains.

Suivant des modes particuliers de réalisation, le procédé comporte l'une ou plusieurs des caractéristiques suivantes, prises indépendamment ou suivant toutes les combinaisons techniquement possibles :

- l'analyse dudit échantillon s'effectue par mise en oeuvre d'un rayonnement électromagnétique dans le domaine spectral du proche infrarouge ;
- l'analyse de l'échantillon s'effectue par une mesure du spectre d'absorbance en transmission de l'échantillon ;
- la préparation de l'échantillon comporte une étape de répartition des épis par couches dans chacune desquelles les épis sont disposés parallèlement les uns aux autres, le sens des épis étant alterné d'une couche adjacente à l'autre ;
- l'analyse dudit échantillon s'effectue par une mesure du spectre de réflectance de l'échantillon éclairé par le rayonnement solaire naturel ;
- l'échantillon est constitué d'épis portés par une céréale sur pied ;
- la teneur en le constituant donné est la teneur en en protéines, en sucres solubles, en cellulose, en azote, en matières grasses, et/ou en soufre.

Suivant des modes particuliers de réalisation, quand il est destiné au calcul de l'indice de nutrition azoté ou à l'estimation de l'état de nutrition, notamment azoté ou soufré, ou de l'état sanitaire, le procédé comporte l'une ou plusieurs des caractéristiques suivantes, prises indépendamment ou suivant toutes les combinaisons techniquement possibles :

- l'analyse dudit échantillon s'effectue par mise en oeuvre d'un rayonnement électromagnétique dans le domaine spectral du proche infrarouge ;
- l'analyse de l'échantillon s'effectue par une mesure du spectre d'absorbance en transmission et/ou en réflexion de l'échantillon ;
- l'analyse de l'échantillon s'effectue par une mesure du spectre de réflectance de l'échantillon éclairé par le rayonnement solaire naturel ;
- la préparation de l'échantillon qui est constitué de parties aériennes des plantes (un ensemble de feuilles et/ou de tiges, et/ou d'épis le cas échéant) qui peuvent être directement au champ ou ramenés du champ;
- la teneur en le constituant donné est la teneur en protéines, en sucres solubles, en cellulose, en azote, et/ou en soufre.

Ainsi, la teneur en protéines déterminée par l'une des variantes de mise en oeuvre du procédé peut être avantageusement utilisée afin de calculer l'indice de nutrition azoté (INN). Celui-ci est en effet calculé à partir de la biomasse sèche par hectare des graminées et du taux de protéines des parties végétatives des graminées considérées.

Ainsi que connu en soi, l'indice de nutrition azoté (INN) permet aux agronomes de déterminer si un apport en agents fertilisants est nécessaire à une croissance satisfaisante de la plante.

Avec le procédé de détermination de la teneur en protéines, le calcul de l'indice de nutrition azotée est plus facilement réalisable, ce qui permet d'améliorer le suivi du cycle végétatif des céréales et d'intervenir plus facilement sur celui-ci.

De même, le procédé selon l'invention peut être avantageusement utilisée dans le but d'estimer l'état de nutrition, notamment azoté ou soufré, l'état sanitaire, le déficit hydrique ou la carence en oligo-éléments afin de contrôler l'apport en agents fertilisants et en produits phytosanitaires tels que des fongicides et/ou des insecticides.

En effet, avec le procédé selon l'invention, des seuils de la teneur en un constituant donné ont pu être déterminés pour différents stades de culture, permettant ainsi le contrôle et une meilleure gestion de la fertilisation, essentiels en terme de rendement et de préservation de l'environnement.

L'invention sera mieux comprise à la lecture de la description qui va suivre, donnée uniquement à titre d'exemple et faite en se référant aux dessins sur lesquels :

- La figure 1 est une vue schématique d'une installation de mesure de la teneur en protéines de grains ;
- La figure 2 est une courbe illustrant la corrélation existant entre la teneur en protéines d'épis de blé et la teneur en protéines des grains qui y sont contenus ;

- La figure 3 est une vue en perspective illustrant un échantillon préparé ; et
- La figure 4 est une vue schématique d'une variante de réalisation d'une installation de mesure de la teneur en protéines de grains utilisable sur des plants sur pied.

Le procédé selon l'invention est destiné à effectuer un suivi d'une culture d'une céréale par mise en oeuvre d'une mesure de la teneur de cette céréale en un constituant déterminé par spectrométrie, avantageusement dans le proche infrarouge. La mesure est effectuée directement sur les plantes en développement post-germination.

A cet effet, suivant un premier mode de mise en oeuvre, le procédé prévoit de mesurer la teneur en un constituant déterminé, notamment en protéines, dans des grains issus de céréales, à partir d'une analyse d'un échantillon constitué d'un ensemble d'épis entiers contenant les grains. La détermination de la teneur des grains en le constituant déterminé comporte une étape de traitement du résultat de l'analyse de l'échantillon pour tenir compte de la présence dans les épis des parties végétales différentes des grains.

L'expression «teneur en un constituant déterminé» désigne la teneur par exemple en protéines ou en azote d'un grain. La teneur d'un produit agricole en un corps déterminé est le rapport de la masse totale du corps déterminé dans le produit sur la masse totale de matière sèche du produit, ou sur la masse total du produit tel quel.

Le terme «épi» désigne les inflorescences constituées d'épillets sessiles (comme les épis de blé) ou d'épillets pédonculés (comme les panicules de l'avoine).

L'expression "plantes en développement post-germination" désigne aussi bien les organes reproducteurs que les organes végétatifs des plantes, mais à un stade ultérieur à la germination.

Dans la suite de la description, le procédé décrit est destiné au calcul de la teneur en protéines.

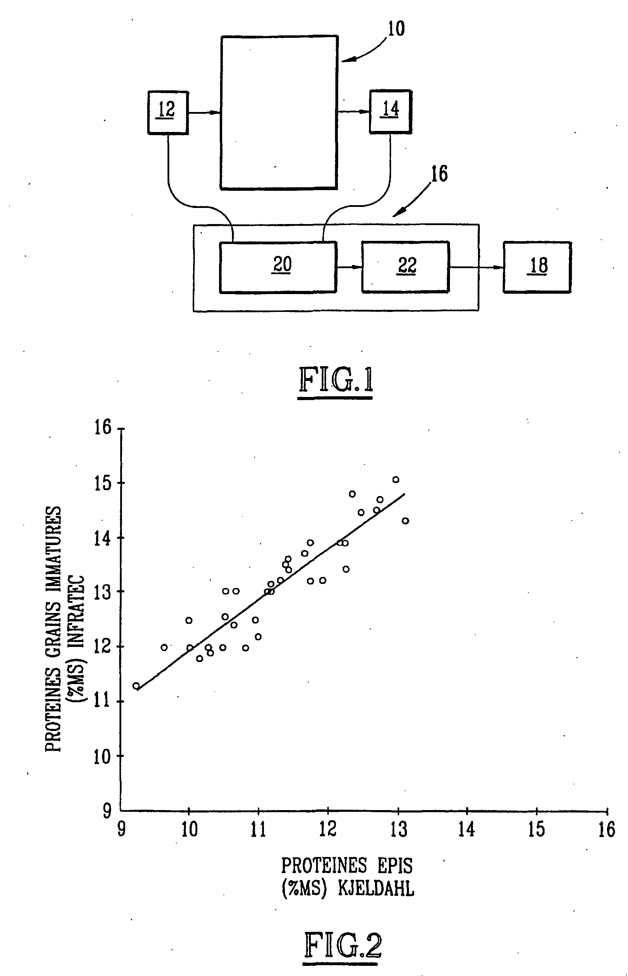

Le procédé de mesure est mis en oeuvre par exemple à l'aide de l'installation représentée sur la figure 1. Celle-ci comporte une chambre 10 de réception de l'échantillon à analyser. De part et d'autre de celle-ci sont disposées une source monochromatique 12 produisant un rayonnement électromagnétique dans le domaine spectral du proche infrarouge et une cellule 14 de réception du rayonnement électromagnétique ayant traversé l'échantillon placé dans la chambre 10.

La source 12 et la cellule 14 sont reliées à une unité centrale de traitement d'informations 16, elle-même connectée à des moyens 18 de mise à disposition des résultats de mesure, par exemple un afficheur.

L'essentiel des éléments de l'installation de la figure 1 est constitué par exemple par l'analyseur de grains «Infratec 1221 » dont l'unité centrale de traitement d'informations a été adaptée pour mettre en oeuvre le procédé de mesure.

Ainsi, la source 12 comprend une lampe au tungstène de 50 W et un réseau de diffraction mobile adapté pour engendrer une lumière monochromatique. Le mouvement du réseau de diffraction assure une illumination de l'échantillon placé dans la chambre 10 avec un rayonnement électromagnétique dont la longueur d'ondes varie de 800 nm à 1100 nm.

La cellule 14 est adaptée pour recevoir le rayonnement électromagnétique ayant traversé les épis constituant l'échantillon placé dans la chambre 10. Le capteur 14 est relié à un module de traitement 20 de l'unité centrale de traitement d'informations 16.

Le module de traitement 20 comporte un convertisseur analogique/numérique relié à des moyens de calcul mettant en oeuvre un algorithme pour déterminer, à partir des signaux recueillis par la sonde 14, la teneur en protéines des épis constituant l'échantillon.

En particulier, et comme connu en soi, le module de traitement 20 met en oeuvre un algorithme permettant de déterminer le spectre d'absorption en transmission de l'échantillon, dans le domaine spectral du proche infrarouge, et d'en déduire la teneur en protéines des épis.

L'information obtenue en sortie du module de traitement 20 correspond par exemple à la valeur mesurée par un analyseur de grains «Infratec 1221 ».

La sortie du module de traitement 20 est reliée à un module de correction 22 adapté pour calculer, à partir de la teneur en protéines des épis déterminée par le module de traitement 20, la teneur en protéines des grains par mise en oeuvre d'un algorithme de correction tenant compte de la présence dans les épis de parties végétales différentes des grains.

La correction appliquée par le module 22 est donnée par la fonction :

$$y = f(x)$$

où y désigne la teneur en protéine des grains et x la teneur en protéine des épis obtenue en sortie du module de traitement 20.

Ainsi, dans le cas des céréales, la fonction peut prendre la forme :

$$y = ax + b.$$

Les constantes a et b, fonction des espèces étudiées, sont déduites de la représentation graphique de la corrélation existant entre la teneur en protéines des épis et la teneur en protéines des grains.

Ainsi, à titre d'exemple, dans le cas du blé, la correction appliquée par le module de correction 22 est donnée par la fonction :

$$y = 0,9208 \ x + 2,7693$$

On a donc a = 0,9208 et b = 2,7693

A titre d'illustration, la figure 2 montre la corrélation existant entre la teneur en protéines des épis et la teneur en protéines des grains contenus dans ces épis. La teneur en protéines des épis a été déterminée par la méthode de Kjeldahl alors que la teneur en protéines des grains a été déterminée par mise en oeuvre de l'analyseur de grains <<Infratec 1221>>. Cette dernière analyse a été réalisée directement sur des grains issus des épis séchés et battus.

L'expérimentation a porté sur trente-sept échantillons dont la teneur en protéines des grains varie de 11 à 15 % de la matière sèche. La droite figurant sur la figure 2 a pour équation :

$$y = 0,9208x + 2,7693.$$

On constate à partir de cette représentation que l'évaluation de la teneur en protéines des grains, obtenue à partir de la teneur en protéines des épis, après correction par le module 22, permet de déterminer une valeur satisfaisante de la teneur en protéines des grains.

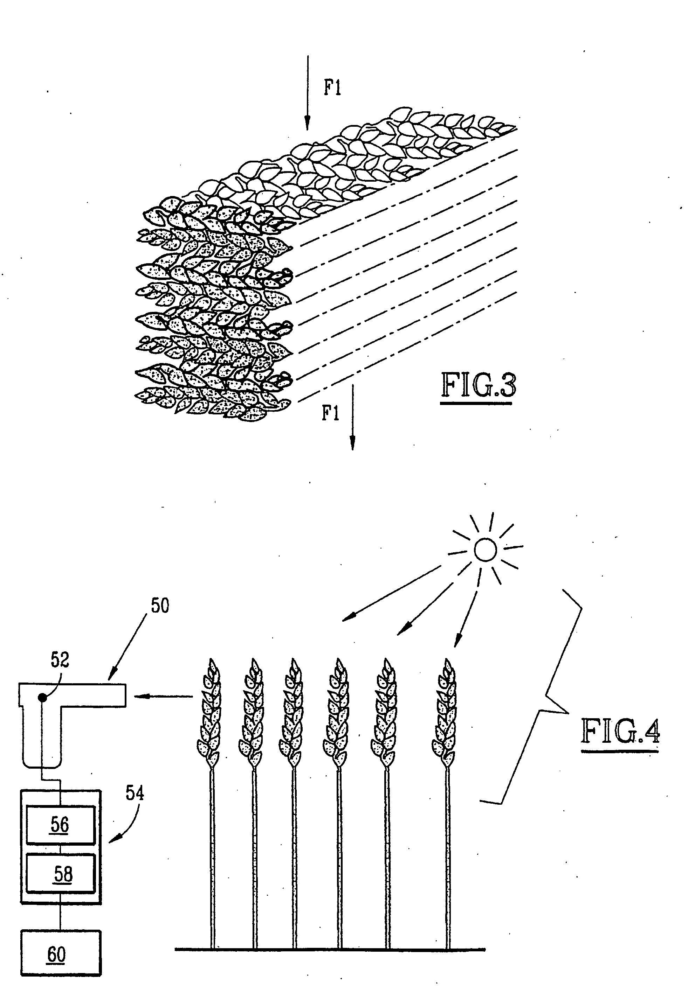

Afin d'obtenir une mesure satisfaisante, l'échantillon placé dans la chambre de mesure 10 est avantageusement préparé et agencé, comme représenté sur la figure 3.

En particulier, les épis entiers, à maturité physiologique, sont séparés de la tige, puis répartis par couches successives. Dans chaque couche, les épis sont disposés parallélement les uns aux autres et dans un même sens. Le sens des épis est alterné d'une couche à l'autre, les épis de deux couches successives étant disposés tête bêche.

L'échantillon ainsi préparé est disposé dans la chambre 10 de sorte que le rayonnement électromagnétique schématisé par la flèche F1 traverse celui-ci perpendiculairement aux plans des couches successives constituant l'échantillon.

Une telle disposition des épis assure une répartition homogène des grains sur tout le volume de l'échantillon, améliorant ainsi la précision de la mesure.

On conçoit que la réalisation de la mesure, directement sur des épis, et non sur les grains, préalablement préparés, allège le protocole opératoire, rendant celui-ci rapide à mettre en oeuvre pour des responsables de silo. De plus, l'expérience montre que les résultats obtenus sont fiables sans qu'il soit nécessaire de procéder à un séchage particulier des épis.

Suivant une variante de mise en oeuvre du procédé de mesure illustrée sur la figure 4, la mesure de la teneur en un constituant déterminé est effectuée alors que les épis sont encore portés par la plante sur pied, directement sur le lieu de culture.

Pour la mise en oeuvre de cette variante du procédé de mesure, l'installation utilisée comporte une sonde portable 50 pouvant être tenue manuellement. Celle-ci comporte un capteur 52 apte à recevoir le rayonnement solaire naturel réfléchi sur les épis portés par les plants en terre.

Le capteur 52 est relié à une unité centrale de traitement d'informations 54 comportant, comme précédemment, un module de traitement 56 et un module de correction 58, lui-même raccordé à un afficheur 60.

Dans ce mode de réalisation, le module de traitement 56 est adapté pour déterminer la teneur en protéines des épis à partir de la valeur de la réflectance du rayonnement solaire naturel sur les épis. La réflectance est déterminée à partir des signaux reçus par le capteur 52.

Comme précédemment, la teneur en protéines des épis obtenue en sortie du module de traitement 56 est corrigée par le module de correction 58 pour tenir compte des parties végétales différentes des grains et ainsi déterminer la teneur en protéines des seuls grains. La valeur ainsi obtenue est rendue accessible à l'utilisateur sur l'écran d'affichage 60.

Dans ce mode de mise en oeuvre également, le protocole opératoire permettant de déterminer la teneur en protéines des grains est simplifié par rapport aux procédés existants nécessitant un traitement préalable lourd des grains.

**Revendications**

1. Procédé de suivi d'une culture de plantes de grandes cultures à épis incluant une mesure de la teneur en un constituant déterminé par spectrométrie dans le domaine du proche infrarouge directement sur les parties aériennes des plantes en développement post-germination **caractérisé en ce que** la mesure par spectrométrie est réalisée sur les épis entiers des plantes afin de déterminer la teneur des grains en un constituant déterminé, et **en ce que** le résultat de l'analyse des épis entiers est traité pour tenir compte de la présence des parties végétales différentes des grains.

2. Procédé selon la revendication 1, **caractérisé en ce que** la mesure de la teneur en un constituant déterminé comporte une mesure du spectre d'absorbance en transmission et/ou en réflexion des épis.

3. Procédé selon la revendication 1 ou 2, **caractérisé en ce que**, avant la mesure par spectrométrie, les épis sont répartis par couches, dans chacune desquelles les épis sont disposés parallèlement les uns aux autres, le sens des épis étant alterné d'une couche adjacente à l'autre.

**4.** Procédé selon la revendication 1, **caractérisé en ce que** la mesure de la teneur en un constituant déterminé comporte une mesure du spectre de réflectance des épis éclairés par le rayonnement solaire naturel.

**5.** Procédé selon la revendication 4, **caractérisé en ce que** les épis sont portés par une céréale sur pied lors de la réalisation de la mesure.

**6.** Procédé selon la revendication 1, **caractérisé en ce que** les échantillons analysés sont portés par des plantes sur pied lors de la réalisation de la mesure.

**7.** Procédé selon l'une quelconque des revendications 1 à 6, **caractérisé en ce que** la mesure par spectrométrie est réalisée sur les parties aériennes des plantes afin de calculer l'indice de nutrition azoté.

**8.** Procédé selon l'une quelconque des revendications 1 à 6, **caractérisé en ce que** la mesure par spectrométrie est réalisée sur les parties aériennes des plantes afin d'estimer l'état de nutrition, notamment azoté ou soufré, le déficit hydrique, la carence en oligo-éléments, ou l'état sanitaire de la culture dans le but de contrôler et de gérer l'apport en agents fertilisants et/ou l'apport en produits phytosanitaires.

**9.** Procédé selon l'une quelconque des revendications précédentes **caractérisé en ce que** la teneur en le constituant donné est la teneur en protéines, en sucres solubles, en cellulose, en azote, en matières grasses, et/ou en soufre.

**10.** Procédé selon l'une quelconque des revendications précédentes. **caractérisé en ce que** la culture de plantes de grandes cultures est une culture d'une céréale.

**11.** Utilisation du procédé selon l'une quelconque des revendications 1 à 6 pour le calcul de l'indice de nutrition azoté.

**12.** Utilisation du procédé selon l'une quelconque des revendications 1 à 6 pour l'estimation de l'état de nutrition, notamment azoté ou soufré, du déficit hydrique, de la carence en oligo-éléments, ou de l'état sanitaire de la culture.

**Claims**

**1.** A method of monitoring growth of field crop plants with ears, including measuring the content of a specific constituent by spectrometry in the near infrared region directly on the aerial parts of the plants in post germination development, **characterized in that** the measurement by spectrometry is effected on the whole ear of the plants, in order to determine the content of a specific constituent of the grains and **in that** the result of the analysis of the whole ears is processed in order to take account of the different vegetable components of the grains.

**2.** A method according to claim 1, **characterized in that** the measurement of the content of a specific constituent comprises measuring the transmission and/or reflection absorption spectrum of the ear.

**3.** A method according to claim 1 or 2, **characterized in that** the ears are distributed in layers before the spectrometric measurement, in each of which the ears are disposed parallel to one another, the sense of the ears alternating from one adjacent layer to the other.

**4.** A method according to claim 1, **characterized in that** the measurement of the content of a specific constituent comprises measuring the reflection spectrum of the ears illuminated by natural sunlight.

**5.** A method according to claim 4, **characterized in that** the ears are carried by a standing cereal during the performance of the measurement.

**6.** A method according to claim 1, **characterized in that** analysed samples are carried by standing plants during the performance of the measurement.

**7.** A method according to any one of claims 1 to 6, **characterized in that** the measurement by spectrometry is effected on the aerial parts of the plants, in order to calculate the nitrogenous nutrition index.

**8.** A method according to any one of claims 1 to 6, **characterized in that** the measurement by spectrometry is effected on the aerial parts of the plants, in order to estimate the nutritional state, especially of nitrogen or sulphur, the moisture deficit, the deficiency of trace elements, the state of health of the growth with the object of controlling and managing the application of fertilising agents and/or the application of phyto-sanitary products.

**9.** A method according to any of the preceding claims, **characterized in that** the content of the given constituent is the content of proteins, soluble sugars, cellulose, nitrogen, fats and/or sulphur.

**10.** A method according to any of the preceding claims, **characterized in that** the growth of field crop plants is growth of a cereal.

**11.** Use of the method according to any of claims 1 to 6 to calculate the nitrogenous nutrition index.

12. Use of the method according to any of claims 1 to 6 to estimate the nutritional state, in particular of nitrogen or sulphur, the moisture deficit, the deficiency of trace elements or the state of health of the growth.

**Patentansprüche**

1. Verfahren zum Überwachen einer Ährenpflanzenkultur in Großanbau, das eine Messung des Gehalts an einem bestimmten Bestandteil durch Spektrometrie im Bereich des nahen Infrarots direkt an den Luftteilen der Pflanzen während der Entwicklung nach der Keimung einschließt, **dadurch gekennzeichnet, daß** die Messung durch Spektrometrie an den ganzen Ähren der Pflanzen vorgenommen wird, um den Gehalt der Körner an einem bestimmten Bestandteil zu bestimmen, und daß das Ergebnis der Analyse der ganzen Ähren behandelt wird, um dem Vorhandensein der verschiedenen Pflanzenteile der Körner Rechnung zu tragen.

2. Verfahren nach Anspruch 1, **dadurch gekennzeichnet, daß** die Messung des Gehalts an einem bestimmten Bestandteil eine Messung des Absorptionsspektrums bei Transmission und/oder Reflexion der Ähren umfaßt.

3. Verfahren nach Anspruch 1 oder 2, **dadurch gekennzeichnet, daß** die Ähren vor der Messung durch Spektrometrie in Schichten verteilt werden, in deren jeder die Ähren parallel zueinander angeordnet werden, wobei der Sinn der Ähren von einer benachbarten Schicht zur anderen abwechselt.

4. Verfahren nach Anspruch 1, **dadurch gekennzeichnet, daß** die Messung des Gehalts an einem bestimmten Bestandteil eine Messung des Reflexionsspektrums der durch natürliche Sonnenstrahlung beleuchteten Ähren umfaßt.

5. Verfahren nach Anspruch 4, **dadurch gekennzeichnet, daß** die Ähren bei der Durchführung der Messung von einer stehenden Getreidepflanze getragen werden.

6. Verfahren nach Anspruch 1, **dadurch gekennzeichnet, daß** die analysierten Proben bei der Durchführung der Messung von stehenden Pflanzen getragen werden.

7. Verfahren nach einem der Ansprüche 1 bis 6, **dadurch gekennzeichnet, daß** die Messung durch Spektrometrie an den Luftteilen der Pflanzen vorgenommen wird, um den Stickstoffernährungsindex zu berechnen.

8. Verfahren nach einem der Ansprüche 1 bis 6, **dadurch gekennzeichnet, daß** die Messung durch Spektrometrie an den Luftteilen der Pflanzen vorgenommen wird, um den Ernährungszustand, insbesondere hinsichtlich Stickstoff oder Schwefel, den Mangel an Spurenelementen oder den Gesundheitszustand der Kultur mit dem Ziel zu ermitteln, die Zufuhr von Düngemitteln und/oder die Zufuhr von Pflanzenschutzmitteln zu überwachen und zu steuern.

9. Verfahren nach einem der vorhergehenden Ansprüche, **dadurch gekennzeichnet, daß** der Gehalt an dem bestimmten Bestandteil der Gehalt an Proteinen, löslichen Zuckern, Celllulose, Stickstoff, Fetten und/oder Schwefel ist.

10. Verfahren nach einem der vorhergehenden Ansprüche, **dadurch gekennzeichnet, daß** die Großanbau-Pflanzenkultur eine Getreidekultur ist.

11. Verwendung des Verfahren nach einem der Ansprüche 1 bis 6 für die Berechnung des Stickstoffernährungsidex.

12. Verwendung des Verfahrens nach einem der Ansprüche 1 bis 6 für die Ermittlung des Ernährungszustands, insbesondere hinsichtlich Stickstoff oder Schwefel, des Wasserdefizits, des Mangels an Spurenelementen oder des Gesundheitszustands der Kultur.

FIG.1

FIG.2

F1

FIG.3

F1

50

52

54

56

58

60

FIG.4